# EUROPÄISCHE PATENTSCHRIFT

(11) **EP 2 138 555 B1**
(45) Veröffentlichungstag und Bekanntmachung des Hinweises auf die Patenterteilung: **11.09.2013**
(21) Anmeldenummer: 09007264.6
(22) Anmeldetag: 30.05.2009
(51) Int. Cl.: B01J 3/00, B01J 19/32, B01J 19/30, C10J 3/57, C10J 3/78, C12P 5/02

(54) **Vorrichtung und Verfahren zur Umsetzung von Biomasse in gasförmige Produkte**
Method and device for converting biomass to gaseous products
Dispositif et procédé de conversion de biomasse dans des produits gazeux

(30) Priorität: 17.06.2008 DE 102008028788
(43) Veröffentlichungstag der Anmeldung: 30.12.2009
(73) Patentinhaber: Karlsruher Institut für Technologie, 76131 Karlsruhe (DE)
(72) Erfinder: Goldacker, Hubert, 76344 Leopoldshafen (DE); Kruse, Andrea, 76646 Bruchsal (DE); Dahmen, Nicolaus, 76646 Bruchsal (DE); Zimmermann, Jens, 06217 Merseburg (DE); Pagel Matthias, 68753 Waghäusel (DE)

(56) Entgegenhaltungen:
- WO-A1-99/64112
- WO-A1-2008/083823
- WO-A2-2005/058472
- GB-A- 1 195 559
- JP-A- 2000 084 576
- US-A1- 2002 084 559

## Beschreibung

Die Erfindung betrifft eine Vorrichtung und ein Verfahren zur Umsetzung von nasser Biomasse, d.h. Biomasse mit einem Wassergehalt von mindestens 50 %, in gasförmige Produkte .

Bei der energetischen Nutzung nasser Biomasse über den Weg der Thermolyse werden häufig überkritisches Wasser mit Drücken und Temperaturen oberhalb des *kritischen Punktes des Wassers* (22,1 MPa und 374 °C) eingesetzt. Während überkritisches Wasser organische Substanzen gut auflöst, fallen anorganische Salze aus und können eine Verstopfung des Reaktors bewirken.

Aus N. Boukis, U. Galla, V. Diem, E, Dinjus, Biomass gasification in supercritical water: First results of the pilot plant, in: A.V. Bridgewater, D.G.B. Boocock, Science in Thermal and Chemical Biomass Conversion, cplpress, Band 2, S. 975, 2006, ist ein schlanker Rohrreaktor bekannt, an dessen Boden sich die im überkritischen Wasser unlöslichen Salze aus der Biomasse sammeln sollen. Da die ausfallenden Salze klebrige bis körnige Konsistenz besitzen, sind nur letztere und nur teilweise durch Vorgänge wie z.B. Sedimentation abtrennbar. Bisher wird die nasse Biomasse in einem vorgeschalteten Wärmetauscher auf Reaktionstemperatur aufgeheizt. Dieses Verfahren ist langsam und setzt die Reaktion bereits im Wärmetauscherrohr in Gang, sobald die notwendige Temperatur erreicht ist.

In einer alternativen Ausgestaltung wird die vorgeheizte, konzentrierte Biomasse zusammen mit überkritischem Wasser in den Reaktor eingespeist. Hierzu ist unter Umständen ein ungünstiges Verhältnis von suspendierter Biomasse zu überkritischem Heizwasser erforderlich. Dadurch wird zwar der Aufheizvorgang beschleunigt und der Beginn der Reaktion in den Reaktor verlegt, jedoch fällt so der Hauptteil der Salze während des Betriebs im Reaktor unkontrolliert und schlecht entfernbar an.

Die US 4,822,497 offenbart einen Druckkessel als Reaktor für die Schadstoffoxidation in überkritischem Wasser, in dem dieselben Probleme bezüglich Salzbildung und -ausscheidung unter Reaktionsbedingungen auftreten. Um die unerwünschte und störende Akkumulation der Salze im Reaktor zu vermeiden, wird der Behälter im Bodenbereich mit einer Wasservorlage betrieben, die durch Quenchen auf einer Temperatur, die deutlich geringer als die kritische Temperatur ist, gehalten wird. Unter diesen Bedingungen lösen sich die Salze aus dem darüber liegenden Reaktionsraum wieder im Wasser. Die Oxidation in überkritischem Wasser ist stark exotherm und daher das Quenchen energetisch vertretbar. Die Vergasung von Biomasse ist demgegenüber endotherm und daher ein Quenchen energetisch nicht vertretbar.

Gemäß Y. Raja, Gasification of waste to produce low-B TU gas by Molten Salt Technique, J. Institution of Engineers India, Band 70, Part T2, S. 15, 1989, werden bei der Biomasse-Vergasung in trockenen Salzschmelzen hohe Umsätze erzeugt, allerdings entsteht hierbei viel Kohlenmonoxid. Zusätzlich muss ein Shift-Konverter nachgeschaltet werden, um Wasserstoff zu erzeugen. Außerdem lässt sich bei Umsetzungen in trockenen Salzschmelzen die Bildung von Holzkohle bzw. Koks nicht vollständig verhindern.

Die DE 202 20 307 U1 offenbart eine Anlage zur Behandlung von fließfähigen Stoffen in überkritischem Wasser, die aus einem zylinderförmigen Reaktor mit Druckleitungen zur Eduktzuführung und Produktableitung besteht, wobei die Produktableitung als Steigrohr ausgebildet ist, das von oben in den Reaktorraum ragt und im unteren Drittel des Reaktors endet, und am unteren Ende des Reaktors ein Sumpfabzug angebracht ist, der an der engsten Stelle liegt und einen Kühler und eine Ventilanordnung zum (dis-)kontinuierlichen Sumpfabzug aufweist.

Die US 6,878,479 B2 offenbart eine Vorrichtung zur direkten Umwandlung von Brennstoffen in elektrische Energie, wobei elektrochemische Zellen, in denen sich jeweils ein geschmolzener Elektrolyten befindet, derart in einer bipolaren, gekippten Konfiguration angeordnet sind, dass der elektrische Widerstand zwischen den Zellen minimal wird.

Aus der US 2005/0066573 A1 ist ein Verfahren zur thermischen Zersetzung von Graphitfasern und Polymerkompositen in einer inerten Atmosphäre bekannt, worin zunächst eine Vielzahl von Graphitfasern, die an ein verkohltes Material gebunden sind, hergestellt und anschließend das verkohlte Material mittels elektrochemischer Oxidation in einer Salzschmelze von den Graphitfasern abgetrennt werden.

Die EP 1 686 192 A1 offenbart ein Verfahren zur Herstellung von Mono- oder Oligosacchariden aus einem Polysaccharid, worin das Polysaccharid in einer hydrothermalen Reaktion in heißem Wasser, dem zuvor Kohlendioxid mittels Druck hinzugefügt wurde, bei einem Druck von 5 bis 100 MPa und einer Temperatur von 140 bis 300 °C hydrolysiert wird.

Aus G. Lee, T. Nunoura, Y. Matsumura und K. Yamamoto, Comparison of the Effects of the addition of NaOH on the decomposition of 2-chlorophenol and phenol in supercritical water and under supercritical water oxidation conditions, J. Supercritical Fluids, Band 24, S. 239-250, 2002, ist bekannt, dass der Einfluss von NaOH auf die Zersetzung von organischen Verbindungen bei der Bestimmung der optimalen Reaktionsbedingungen und des optimalen Reaktordesigns berücksichtigt werden muss.

D.D. MacDonald und L.B. Kriksunov beschreiben in ihrem Übersichtsartikel Probing the chemical and electrochemical properties of the SCWO system, Electrochimica Acta, Band 47, S. 775-790, 2001, Vorteile des und Hindernisse beim Einsatz von überkritischer Oxidation in Wasser (*supercritical water oxidation,* SCWO). Die Vorteile von SCWO gegenüber Verbrennung liegen im Einsatz von geschlossenen Zyklen bei der Durchführung der Reaktion und in der höheren Zersetzungseffizienz. Letztere wird jedoch durch eine höhere Korrosivität gegenüber üblichen Materialien erkauft.

Aus K. Pripopsky, B. Wellig und Ph.R. von Rohr, SCWO of salt containing artificial wastewater using a transpiring wall reactor: Experimental results, J. Supercritical Fluids, Band 40, S. 246-257, 2007, über Science Direct verfügbar ab 07.07. 2007, ist ein Reaktoraufbau bekannt, der über zwei verschiedene teildurchlässige Wandelemente verfügt, womit sich das Problem des Reaktorfaulens und der Verstopfung durch Salzausfällungen vermeiden lassen.

M. Hodes, P.A. Marrone, G.T. Hong, K.A. Smith und J.W. Tester beschreiben in Salt precipitation and scale control in supercritical water oxidation, Part A: Fundamentals and research, J. Supercritical Fluids, Band 29, S. 265-288, 2004, die Prinzipien der Salzausfällung die Skalierung bei höheren Temperaturen und Drücken, Phasendiagramme des Salz-Wasser-Systems und sich hieraus ergebende Phänomene.

In Z. Sun, F. Takahashi, Y. Odaka, k. Fukushi, Y. Oshima und K. Yamamoto, Effects of potassium alkalis and sodium alkalis on the dechlorination of o-chlorophenol in supercritical water, Chemosphere, Band 66, S. 151-157, 2007, online ab dem 29. 06.2006 verfügbar, wird der Einfluss von Kalium- und Natrium-Alkalien auf die Entchlorinierung von o-Chlorophenol in überkritischem Wasser beschrieben.

M.D. Bermejo, A. Martin, L.J. Florusse, C.J. Peters und M.J. Cocero beschreiben in The influence of Na2SO4 on the CO2 solubility in water at high pressure, Fluid Phase Equilibria, Band 238, S. 220-228, 2005, die effektive Zersetzung von Abfall am Beispiel von Na₂SO₄ in überkritischem Wasser. Hierbei wurden Zersetzungsraten von über 99 % bei Aufenthaltszeiten von weniger als 1 Minute beobachtet.

Aus M.D. Bermejo, A. Martin, L.J. Florusse, C.J. Peters und M.J. Cocero, Bubble points of the systems isopropanol-water, isopropanol-water-sodium acetate and isopropanol-water-sodium oleate at high pressure, Fluid Phase Equilibria, Band 244, S. 78-85, 2006, ist bekannt, dass die Oxidation in überkritischem Wasser eine wirkungsvolle Technik für die Zersetzung von organischem Abfall mit hohem Ausbeute darstellt. Sobald genügend Kationen vorhanden sind, fallen vorhandene Heteroatome in Form von Salzen aus und lassen sich schließlich wiedergewinnen.

Die JP 2000 84576 A offenbart eine Vorrichtung, aufweisend einen Reaktor, eine Heizung zur Aufheizung des Reaktors und seines Inhalts, ein Einspeiserohr, ein Auslassrohr zur Ausleitung von gasförmigen Produkten aus dem Reaktor und einen Abzug im Boden für Teile der Salzschmelze. Bei dieser Vorrichtung handelt es sich um einen Sedimentationsreaktor.

Aus der WO 2005/058472 A2 ist es bekannt, eine Packung in einen Reaktor für überkritische Bedingungen einzufügen, um die gleichmäßige Verteilung der Reaktanden zu erhöhen, um auf diese Weise die Homogenität der erzeugten Partikel zu erhöhen.

Ausgehend hiervon ist es die Aufgabe der vorliegenden Erfindung, eine Vorrichtung und ein Verfahren vorzuschlagen, die die genannten Nachteile und Einschränkungen nicht aufweisen.

Insbesondere sollen die thermolytische Zersetzung der Biomasse und die Entstehung zusätzlicher Salze aus der Biomasse erst innerhalb des Reaktors erfolgen, damit die zusätzlichen Salze direkt am Ort ihrer Entstehung gebunden werden können.

Weiterhin soll die Vorrichtung derart ausgestaltet sein, dass sie eine kontinuierliche Betriebsführung ermöglicht.

Diese Aufgabe wird im Hinblick auf die Vorrichtung durch die Schritte des Anspruchs 1 und im Hinblick auf das Verfahren durch die Merkmale des Anspruchs 5.

Die Unteransprüche beschreiben jeweils vorteilhafte Ausgestaltungen.

Eine erfindungsgemäße Vorrichtung zur Umsetzung von Biomasse mit einem Wassergehalt von mindestens 50 %, enthält mindestens die folgenden Bestandteile:
- einen Druckreaktor (Autoklaven), der bei einem Druck oberhalb des kritischen Drucks des Wassers, bevorzugt oberhalb von 22,5 MPa betrieben werden kann und der sich zur Aufnahme einer Salzschmelze eignet,
- eine Packung aus einem oder mehreren Füllkörpern, die den Reaktor ganz oder teilweise, zumindest zum überwiegenden Teil ausfüllen, zur Erhöhung der Austauschfläche,
- eine Heizung, die zur Aufheizung des Reaktors und seines Inhalts, insbesondere der sich hierin befindlichen Salzschmelze auf die Reaktionstemperatur, die zur Umsetzung der Biomasse in gasförmige Produkte erforderlich ist, geeignet ist,
- ein erstes Einspeiserohr zur Zuführung von Wasser und Salzlösung in den Reaktor,
- ein zweites Einspeiserohr zur Zuführung der Biomasse in den Reaktor,
- ein Auslassrohr zur Ausleitung der gasförmigen Reaktionsprodukte aus dem Reaktor und
- einen Abzug im Boden des Reaktors zum Entnehmen von Teilen der Salzschmelze, die mit den weiteren aus der Biomasse heraus gelangten Salzen angereichert ist, aus dem Reaktor.

Vorzugsweise kommen für die Packung Füllkörper in Frage, wie sie für eine Extraktion oder Rektifikation geeignet sind, wozu bevorzugt Ring-, Sattel- oder Gitter-Füllkörper, strukturierte Blechpackungen, Berl-Sattel, Braunschweiger Wendeln, Drahtspiralen, Glockenböden, Igel, Maschinendrahtringe, oder Mini-Kaskaden-Ringe gehören. Derartige Füllkörper sind im Handel insbesondere unter den Marken Envipac®, Hacketten®, Hel-X®, Hiflow®-Ringe oder -Sattel, IMTP®, Intalox®-Sattel, Interpack®, Kühni®-Strukturpackungen, Leva-Ringe®, NOR-PAK®, Montz®-Strukturpackungen, Novalox®-Sattel, Pall-Ringe®, RALU-PAK®, Raschig®-Austauschpackungen oder -Ringe, Snowflakes®, Sulzer® Gewebe-, Lamellen- oder Strukturpackungen, Super-Sattel® oder Super-Torus-Sattel®, Telleretten®, Top-Paks®, VFF-Net-Balls®, V-Paks® oder VSP® erhältlich.

Das Material, aus dem der Füllkörper besteht, muss hierbei den im Reaktor herrschenden Bedingungen widerstehen können. Hierfür eignen sich in erster Linie vorzugsweise Metalle, Metalloxide, bevorzugt TiO₂ oder Al₂O₃, Legierungen, bevorzugt Nickelbasis-Legierungen, oder Kohlenstoff.

Das erfindungsgemäße Verfahren wird in einem Druckreaktor bei einem Druck oberhalb des kritischen Drucks des Wassers, bevorzugt oberhalb von 22,5 MPa, durchgeführt, der in einem ersten Schritt a) vorzugsweise von oben mit einer Salzlösung, die das Salz enthält, das die hydrothermale Schmelze bilden soll, bevorzugt über ein erstes Einspeiserohr, gefüllt wird.

Der Schmelzpunkt des für die Salzschmelze eingesetzten Salzes oder der hierfür eingesetzten Salzmischung muss unterhalb der Reaktionstemperatur liegen, bei der die Biomasse in ein gasförmiges Produkt umgesetzt wird. Die Reaktionstemperatur beträgt in der Regel 550 °C bis 700 °C.

Als Salze eignen sich vorzugsweise solche Salze, die wasserhaltige Schmelzen mit niedrigen Schmelzpunkten bilden. Besonders bevorzugt sind Alkalisalze, insbesondere Alkalinitrate, -hydroxide, -carbonate, -phosphate, -borate, -hydrogencarbonate oder -halogenide wie -chlorate oder -perchlorate, Ammoniumsalze, insbesondere -halogenide, -sulfat, -carbonat oder -hydrogencarbonat, oder Salze von Zink, Magnesium, Calcium oder Eisen, insbesondere Halogenide, aber auch Zinkchlorat oder -perchlorat oder Eisenhydrate.

Beispiele für geeignete Salzmischungen sind eine 50:50-Mischung aus NaNO₃ und KNO₃ (Schmelzpunkt 223 °C) oder eine 56:44-Mischung aus LiNO₃ und KNO₃ (Schmelzpunkt 125 °C). Ein weiteres Beispiel für ein geeignetes Salz ist KOH mit einem Schmelzpunkt von 360 °C.

Die für die Salzschmelze eingesetzten Salze bzw. Salzmischungen besitzen darüber hinaus vorzugsweise die folgenden Eigenschaften:
- Sie weisen eine ausreichende thermische Stabilität für Temperaturen bis zu 700 °C bei Betriebsdruck auf.
- Sie besitzen eine möglichst geringe Korrosivität.
- Der Schmelzpunkt ändert sich durch den Eintrag von weiteren Salzen aus der Umsetzung der Biomasse nur insoweit, dass die Verfahrensführung nicht wesentlich beeinträchtigt wird.
- Die Viskosität stellt die Fließfähigkeit der Salzschmelze im Reaktor sicher.
- Die spezifische Wärme erlaubt die rasche Aufheizung der vorgeheizten Biomasse durch den Kontakt mit der Salzschmelze.

Die sich im Reaktor befindliche Salzschmelze wird gemäß Schritt b) von außen auf die Reaktionstemperatur, die zur Umsetzung der Biomasse in gasförmige Produkte erforderlich ist, aufgeheizt. Das Aufheizen erfolgt z.B. durch Rauchgase.

Durch die erhöhte Temperatur verteilt sich die Salzschmelze auf der Packung und fließt vorzugsweise nach unten. Die Salzschmelze hat eine deutlich höhere Dichte als Wasser unter Reaktionsbedingungen. Dabei besitzt die Schmelze eine große Neigung, an Wandungen oder Einbauten, wie sie die Packung bietet, entlang zu kriechen.

Die wässrige Biomasse wird nun gemäß Schritt c), vorzugsweise über ein zweites Einspeiserohr, in den Reaktor eingespeist, wodurch die Biomasse auf die Reaktionstemperatur aufgeheizt wird und in mehr oder weniger abgebauter Form auf der Packung aus dem mindestens einen Füllkörper in Kontakt mit der dort verteilten hydrothermalen Salzschmelze kommt. Hierbei wirkt die Schmelze zum einen als Katalysator für die Bildung der gasförmigen Produkte, wodurch die Thermolyse der Biomasse innerhalb kürzester Zeit in Gang kommt und der Biomassekohlenstoff in geeigneter Form zur Wasserspaltung (sog. *Wassergas-Shift-Reaktion*) zur Verfügung steht. Zum anderen nimmt die Salzschmelze die bei der Thermolyse der Biomasse freiwerdenden weiteren Salze auf.

In einer bevorzugten Ausgestaltung wird die Biomasse während Schritt c) zunächst in einem Wärmetauscher auf eine Vorheiztemperatur, bei der noch keine störende Zersetzung der organischen Verbindungen einsetzt, aufgeheizt.

Gemäß Schritt d) wird vorzugsweise kontinuierlich ein mit den aus der Biomasse stammenden Salzen angereicherter Teil der Salzschmelze über einen Abzug am Boden des Reaktors abgezogen. Die entnommene Salzmenge wird durch frische Salzmischung ersetzt.

Gemäß Schritt e) werden die gasförmigen Produkte über ein Auslassrohr, das sich bevorzugt oben am Reaktor befindet, entnommen.

Das erfindungsgemäße Verfahren weist insbesondere die folgenden Vorteile auf:
- Die thermolytische Zersetzung der Biomasse und damit die Entstehung der zusätzlichen Salze aus der Biomasse setzen erst oberhalb der Vorheiztemperatur innerhalb des Reaktors ein, so dass die Salze direkt am Ort der Entstehung gebunden werden können.
- Die Bindung der Salze aus der Thermolyse der Biomasse erfolgt in der vorgelegten Salzschmelze, die gleichzeitig als Wärmequelle zum Aufheizen der vorgeheizte Biomasse auf die Reaktionstemperatur dient.
- Umfasst die Salzschmelze ein Alkalisalz oder eine Salzmischung, bei der mindestens eine Komponente ein Alkalisalz ist, so kann während der Erwärmung in der Salzschmelze von der Vorheiz- auf Reaktionstemperatur zusätzlich die Reaktivität der Biomasse derart erhöht werden, dass ein möglichst vollständiger Umsatz der in der Biomasse enthaltenen gebundenen Kohlenstoffatome zu CO₂ und CH₄ erreicht wird.
- Durch die kontinuierliche Betriebsführung werden Störungen des Systems insbesondere durch Druckschwankungen vermieden.
- Durch das Gegenstromprinzip innerhalb der Packung werden die Salze effizient aufgenommen und auch Biomassen mit hohen Salzfrachten können bearbeitet werden, da *frische* Schmelze auf eine von Salzen *abgereicherte* Lösung trifft und ihr noch die letzten Salze entzieht. Dadurch lassen sich die gebundenen Salze ohne Beeinträchtigung des Betriebs aus dem Reaktor entfernen.
- Die Füllkörper in der Packung erhöhen die Austauschfläche zwischen der hydrothermalen Schmelze und der biomassehaltigen Lösung.

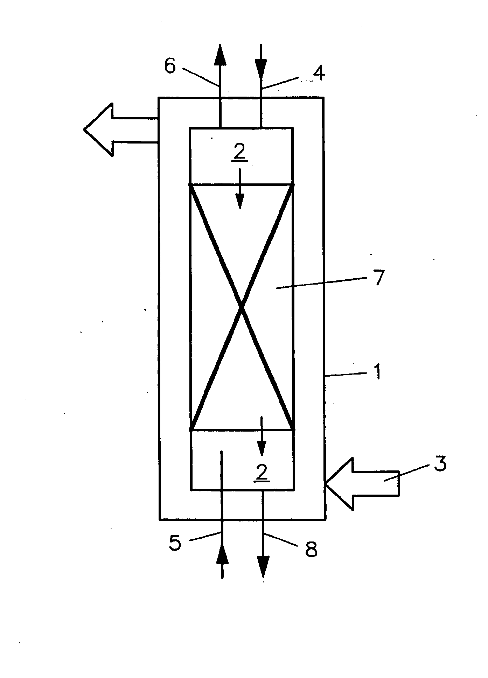

Die Erfindung wird im Folgenden anhand eines Ausführungsbeispiels und der **Figur** näher erläutert. In der Figur ist ein Ausführungsbeispiel für eine erfindungsgemäße Vorrichtung zur Durchführung des erfindungsgemäßen Verfahrens dargestellt.

Ein Hockdruckreaktor (Autoklav) **1** wird von oben über ein erstes Einspeiserohr **4** mit einer Salzschmelze **2** befüllt, die zur Erzeugung einer hydrothermalen Schmelze vorgesehen ist und die mittels einer Heizung **3** aufgeheizt wird. Der Reaktor **1** ist in diesem Beispiel zum überwiegenden Teil mit einer Packung **7** aus den Füllkörpern Raschig®-Ringen oder Braunschweiger Wendeln gefüllt. Durch die erhöhte Temperatur bildet sich die hydrothermale Schmelze, die eine deutlich höhere Dichte als Wasser unter Reaktionsbedingungen aufweist und sich daher auf die Packung **7** verteilt und nach unten fließt.

Von unten wird über ein zweites Einspeiserohr **5** eine Mischung aus Biomasse und Wasser, die sich hier auf einer Vorheiztemperatur, bei der noch keine störende Zersetzung der organischen Verbindungen in der Biomasse auftritt, befindet, in den Reaktor **1** eingeführt.

Auf den Füllkörpern der Packung **7** findet ein intensiver Kontakt zwischen der hydrothermalen Schmelze und mehr oder weniger abgebauter Biomasse statt. Hierbei wirkt die Salzschmelze zum einen als Katalysator für die Gasbildung, zum anderen nimmt sie Salze aus der Biomasse auf.

Die während des vorliegenden, kontinuierlich ablaufenden Verfahrens gebildeten Gase verlassen, in Wasser gelöst, über ein Auslassrohr **6,** das sich hier am Kopf des Reaktors **1** befindet, den Reaktor **1.** Die Salzschmelze **2,** die mit den weiteren aus der Biomasse heraus gelangten Salzen angereichert ist, fließt ebenfalls kontinuierlich durch den Auslass **8** am Boden des Reaktors **1** heraus.

## Patentansprüche

1. Vorrichtung zur Umsetzung von Biomasse, die einen Wassergehalt von mindestens 50 % aufweist, aufweisend
- einen Reaktor (1), der zumindest teilweise mit einer Packung (7) aus mindestens einem Füllkörper ausgestattet ist, zur Aufnahme von überkritischem Wasser und einer Salzschmelze (2),
- eine Heizung (3) zur Aufheizung des Reaktors (1) und seines Inhalts,
- ein erstes Einspeiserohr (4) zur Zuführung von Wasser und Salzlösung von oben in den Reaktor (1),
- ein zweites Einspeiserohr (5) zur Zuführung der Biomasse von unten in den Reaktor (1),
- ein Auslassrohr (6) am Kopf des Reaktors (1) zur Ausleitung von gasförmigen Produkten aus dem Reaktor (1) und
- einen Abzug (8) im Boden des Reaktors (1) für Teile der Salzschmelze (2).

2. Vorrichtung nach Anspruch 1, wobei die Füllkörper in der Packung (7) die Austauschfläche zwischen der hydrothermalen Schmelze und der wässrigen Biomasse erhöhen.

3. Vorrichtung nach Anspruch 2, wobei die Packung (7) Ring-, Sattel- oder Gitter-Füllkörper, strukturierte Blechpackungen, Berl-Sattel, Braunschweiger Wendeln, Drahtspiralen, Glockenböden, Igel, Maschinendrahtringe oder Mini-Kaskaden-Ringe enthält.

4. Vorrichtung nach einem der Ansprüche 1 bis 3, wobei die Packung (7) aus einem Metall, einem Metalloxid, einer Legierung oder aus Kohlenstoff besteht.

5. Verfahren zur Umsetzung von Biomasse, die einen Wassergehalt von mindestens 50 % aufweist, das oberhalb des kritischen Drucks des Wassers durchgeführt wird, aufweisend die Schritte
a) Befüllen eines Reaktors (1) von oben mit einer Salzschmelze (2), wobei die Salzschmelze (2) aus einem Salz oder einer Salzmischung mit einem Schmelzpunkt unterhalb einer Reaktionstemperatur, die zur Umsetzung der Biomasse in gasförmige Produkte erforderlich ist, besteht,
b) Aufheizen der Salzschmelze (2), so dass sich die Schmelze auf einer, sich im Reaktor (1) befindlichen Packung (7) aus dem mindestens einen Füllkörper verteilt,
c) Einspeisen von wässriger Biomasse von unten in den Reaktor (1), die auf die Reaktionstemperatur aufgeheizt wird und auf der Packung (7) aus dem mindestens einen Füllkörper in Kontakt mit der Salzschmelze (2) kommt, wodurch die Salzschmelze als Katalysator für die Gasbildung wirkt, so dass die Umsetzung der Biomasse in gasförmige Produkte eintritt, und durch die erfolgte Umsetzung weitere Salze aus der Biomasse heraus in der Salzschmelze (2) gelangen,
d) Entnehmen von Teilen der Salzschmelze (2), die mit den weiteren aus der Biomasse heraus gelangten Salzen angereichert ist, aus dem Boden des Reaktors (1) und Ersetzen der entnommenen Menge an Salzschmelze (2) durch eine frische Salzlösung, und
e) Entnehmen der gasförmigen Produkte über ein Auslassrohr (6), das sich am Kopf des Reaktors 1 befindet, aus dem Reaktor (1).

6. Verfahren nach Anspruch 5, wobei ein Alkalisalz als Salz oder als Bestandteil der Salzmischung eingesetzt wird.

7. Verfahren nach Anspruch 6, wobei ein Alkalisalz, ein Ammoniumsalz oder ein Zink-, Magnesium-, Calcium- oder Eisensalz als Salz oder als Bestandteil der Salzmischung eingesetzt wird.

8. Verfahren nach Anspruch 7, wobei ein Halogenid als Salz oder als Bestandteil der Salzmischung eingesetzt wird.

9. Verfahren nach einem der Ansprüche 5 bis 8, wobei das Entnehmen von Teilen der Salzschmelze (2) kontinuierlich erfolgt.

## Claims

1. Device for converting biomass with a water content of at least 50%, comprising
- a reactor (1) filled at least partially with a packing (7) including at least one filler body for accommodating supercritical water and molten salt (2),
- a heater (3) arranged to heat up the reactor (1) and its content,
- a first feeding pipe (4) to feed water and salt solution from above into the reactor (1),
- a second feed pipe (5) to feed to the biomass from below into the reactor (1),
- a discharge pipe (6) at the head of the reactor (1) to discharge gaseous products from the reactor (1) and
- an outlet (8) in the bottom of the reactor (1) for portions of the molten salt (2).

2. Device according to claim 1, wherein the filler bodies in the packing (7) increase the exchange surface between the hydrothermal melt and the watery biomass.

3. Device according to claim 2, wherein the packing (7) contains ring, saddle or lattice filler bodies, structured sheet metal packagings, Berl saddles, Brunswick coils, wire spirals, bell bottoms, hedgehogs, machine wire rings or mini-cascade rings.

4. Device according to any one of claims 1 to 3, wherein the packing (7) consists of a metal, a metal oxide, an alloy, or of carbon.

5. Method for converting biomass with a water content of at least 50% which is implemented above the critical water pressure, comprising the following steps
a) filling a reactor (1) from above with molten salt (2), wherein the molten salt (2) consists of one of a salt or a salt mixture having a melting point below a reaction temperature required for converting the biomass to gaseous products,
b) heating the molten salt (2) so that the melt spreads out inside the reactor (1) over a packing (7) with the at least one filler body,
c) feeding a watery biomass from below into the reactor (1), where the biomass is then heated up to the reaction temperature and comes in contact with the molten salt (2) on the packing (7) with the at least one filler body, whereby the molten salt acts as a catalyst for the gas production, thus resulting in the conversion of the biomass to gaseous products and causing additional salts from the biomass to be bonded to the molten salt (2),
d) removing portions of the molten salt (2) enriched with additional salts from the biomass from the bottom of the reactor (1) and replacing the removed portion of molten salt (2) with a fresh salt solution, and
e) removing the gaseous products via a discharge pipe (6), which is located at the head of the reactor 1, from the reactor (1).

6. Method according to claim 5, wherein an alkali salt is used as a salt or as a component part of the salt mixture.

7. Method according to claim 6, wherein an alkali salt, an ammonium salt or a zinc, magnesium, calcium or iron salt is used as a salt or as a component part of the salt mixture.

8. Method according to claim 7, wherein a halogenide is used as a salt or as a component part of the salt mixture.

9. Method according to any one of claims 5 to 8, wherein portions of the molten salt (2) are continuously removed.

## Revendications

1. Dispositif de conversion de biomasse renfermant une teneur en eau d'au moins 50 % comprenant :
- un réacteur (1) qui est au moins partiellement équipé d'un garnissage (7) constitué par au moins une charge, pour la réception d'eau sur-critique et d'une masse de sel fondu (2),
- des organes de chauffage (3) pour permettre de chauffer le réacteur (1) et son contenu,
- un premier tube d'alimentation (4) pour permettre d'amenée l'eau et d'une solution saline par le haut dans le réacteur (1),
- un second tube d'alimentation (5) pour permettre l'amenée de la biomasse par le bas dans le réacteur (1),
- un tube de sortie (6) situé à la tête du réacteur (1) pour permettre d'évacuer les produits gazeux du réacteur (1), et
- une évacuation (8) située au fond du réacteur (1) pour permettre l'évacuation de parties de la masse de sel fondu (2).

2. Dispositif conforme à la revendication 1,
dans lequel
la charge du garnissage (7) augmente la surface d'échange entre la masse fondue hydrothermale et la biomasse aqueuse.

3. Dispositif conforme à la revendication 2,
dans lequel
le garnissage (7) renferme des corps annulaires, coudés ou en forme de grille, des paquets de tôles structurés, des corps de Berl, des spirales de fils, des ressorts Braunschweig, des fonds de cloches, des peignes circulaires, des anneaux de fils métalliques ou des anneaux en mini cascades.

4. Dispositif conforme à l'une des revendications 1 à 3,
dans lequel
le garnissage (7) est constitué d'un métal, d'un oxyde métallique, d'un alliage ou est réalisé en carbone.

5. Procédé de conversion de biomasse ayant une teneur en eau d'au moins 50 % comprenant qui est mis en oeuvre au-dessus de la pression critique de l'eau comportant les étapes consistant à :
a) remplir un réacteur (1), par le haut, avec une masse de sel fondu (2), cette masse de sel fondu (2) étant constituée d'un sel ou d'un mélange de sels ayant un point de fusion situé au-dessous de la température de réaction nécessaire pour la conversion de la biomasse en produits gazeux,
b) chauffer la masse de sel fondu (2) de sorte que cette masse se répartisse sur un garnissage (7), constitué d'au moins une charge se trouvant dans le réacteur (1),
c) introduire par le bas dans le réacteur (1) la biomasse aqueuse qui est chauffée à la température de réaction et vient en contact sur le garnissage (7) constitué d'au moins une charge avec la masse de sel fondu (2), cette masse de sel fondu faisant office de catalyseur pour la formation de gaz de sorte que la conversion de la biomasse en produits gazeux se produise et que, suite à la conversion ainsi effectuée, d'autres sels provenant de la biomasse parviennent dans la masse de sel fondu (2),
d) prélèvement de parties de la masse de sel fondu (2) qui est enrichie par les autres sels provenant de la biomasse, à partir du fond du réacteur (1) et remplacement de la quantité de masse de sel fondu (2) ainsi prélevée par une solution saline fraîche, et
e) prélèvement du réacteur (1) des produits gazeux par un tube de sortie (6) qui se trouve à la tête du réacteur (1).

6. Procédé conforme à la revendication 5,
selon lequel
on met en oeuvre un sel alcalin en tant que sel ou constituant du mélange de sels.

7. Procédé conforme à la revendication 6,
selon lequel
on met en oeuvre, en tant que sel ou en tant que constituant du mélange de sels, un sel alcalin, un sel d'ammonium ou un sel de zinc, de magnésium, de calcium ou de fer.

8. Procédé conforme à la revendication 7,
selon lequel
on met en oeuvre un halogénure en tant que sel on constituant du mélange de sels

9. Procédé conforme à l'une des revendications 5 à 8,
selon lequel
le prélèvement de parties de la masse de sel fondu (2) est effectué en continu.
